# EUROPEAN PATENT SPECIFICATION

(11) **EP 4 531 774 B1**
(45) Date of publication and mention of the grant of the patent: **17.09.2025**
(21) Application number: 24739688.0
(22) Date of filing: 07.06.2024
(51) Int. Cl.: A61F 9/00, A61B 17/02

(54) **DEVICE FOR EYE POSITIONING AND IMMOBILIZATION DURING OPHTHALMOLOGICAL SURGERY**
VORRICHTUNG ZUR AUGENPOSITIONIERUNG UND IMMOBILISIERUNG WÄHREND EINER AUGENCHIRURGIE
DISPOSITIF DE POSITIONNEMENT ET D'IMMOBILISATION DE L'OEIL PENDANT UNE CHIRURGIE OPHTALMOLOGIQUE

(30) Priority: 07.06.2023 PT 2023118711; 16.08.2023 EP 23191775
(43) Date of publication of application: 09.04.2025
(73) Proprietor: Gabriel Morgado Serviços Médicos, Lda, 4460-189 Senhora da Hora (PT)
(72) Inventor: MORGADO SANTOS, Gabriel José, 4460-189 Senhora da Hora (PT)
(74) Representative: Pereira da Cruz, Joao
(86) International application number: PCT/IB2024/055611
(87) International publication number: WO 2024/252363

(56) References cited:
- EP-A1- 0 156 218
- WO-A1-2020/080792
- US-A- 5 341 798

## Description

### TECHNICAL FIELD

The present disclosure relates to an eye globe positioning and immobilization device for use during ophthalmological surgery, in particular during glaucoma, strabismus and pterygium surgery.

### BACKGROUND

When ophthalmological surgery procedures require eye positioning and immobilization, there is a need for effective, safe, practical means for positioning and immobilizing the eye during said procedures.

Examples of immobilization devices used for cornea refractive surgery and intravitreal injections are in the following documents.

Document EP1147754A1 discloses a suction ring which immobilizes and imparts vacuum pressure to the eyeball through an orifice, the vacuum pressure being distributed simultaneously from the ring through a peripheral channel situated in the same.

As another example, document CN112515846A discloses an intraocular injection auxiliary device.

In glaucoma surgery (trabeculectomy, deep sclerectomy, tube shunt devices, or other glaucoma devices) there is a need for eye positioning to expose a surface area of sclera, subtenon's space, periocular space, where surgeries are performed or devices are implanted.

The same need is necessary for strabismus and pterygium surgery, for example. Current methods are traction sutures that pass through the cornea or rectus muscles, that are secured by tightening a forceps to periocular drapes or using tape. These methods are not as effective, safe, and practical as ophthalmological surgeries require. Sutures fixation often slide, surgical area exposition is often limited, there is no adjustability and often there is need of human hand to help circumvent these limitations. There is thus a need for a device that addresses these problems.

One of the problems to be solved may be seen as the need in ophthalmic surgery for eye positioning, eye immobilization and eye position adjustability, in order to expose areas of the eye where certain surgeries are performed, such as areas behind the limbus, which are only exposed when the eye is positioned in extreme gaze positions. This is a problem that has more than 60 years. The need for its resolution has steadily increased as the number and different types of ophthalmic surgeries have also increased. To perform these surgeries, two conditions must be met:
1. The eyelids must be spread apart, to give access to the eye, which is efficiently 2. accomplished with an eyelid speculum / retractor with two opposite arms and a pivotal point. This gives access to the eye. There are several types of this devices. The eye remains in primary position, this is with the gaze straight ahead such as in devices US7985180B2, US5341798A, CN202714969U, this exposure of the eye is sufficient to perform ocular surgeries that occur anterior to the corneal limbus such as cataract and refractive surgery, but insufficient to perform surgeries that occur posterior to the limbus. In some cases additional features were developed on these devices, US20070179346A1 describes an eyelid speculum with an aspiration system to avoid fluid accumulation in the conjunctiva fornix and WO2020080792A1 has a complex system for adjusting the thread on adjustable strabismus surgery. In the prior art, the most efficient devices to spread apart the eyelids are eyelid specula, attempts were performed with other devices, such as devices that were mainly designed as wound retractors, such as EP0156218A1. This document discloses a retractor with an adhesive pad and a bowed portion extending therefrom, the bowed portion being sharply turned back at the free end thereby defining a hook means with two sharp tangs for impaling the tissue which is to be retracted. Its absence of efficacy and even danger to the delicate structures of the eye and adnexa, rendered them unusable in the field of ophthalmology, even for retracting eyelids. For clarity purposes devices destined to spread apart / retract the eyelids are not the scope of this invention, such as the previous referred ones;
2. To perform eye surgeries in ocular areas behind the limbus, the eye must be forced, in/to extreme gaze positions, to expose these areas, with traction sutures such as in the present disclosure.

The present disclosure does not pertain to an eyelid retractor, i.e. devices intended for the mobilization or separation of the eyelids. Instead, it pertains to the field of eye (eye globe, eyeball) positioning, eye immobilization, and eye position adjustability, in order to expose areas of the eye where certain eye surgeries are performed. The claimed device is directed to the eye globe itself, for rotating, positioning it and immobilizing it in a desired position.

The disclosure is thus directed to the second point, as it creates a solid anchor point for the eye traction sutures to be secured (avoiding suture slippage that commonly occurs in the state of art practice that is using tape or a forceps to secure the sutures), it allows adjustability of the eye position, enhancing gaze positions, that results in a broader exposed surface of the eye, where the surgeries can be performed.

Prior art devices are mostly directed to the eyelids, not the eye itself, and they cannot in any realistic way work on the second condition, this is positioning the eye in/to extreme gaze positions, and expose areas where surgeries are performed, that is the subject matter of the present disclosure.

These facts are disclosed to illustrate the technical problem addressed by the present disclosure.

### GENERAL DESCRIPTION

The invention relates to an ophthalmic surgical device for eye globe positioning and immobilization using eye globe traction suture or sutures and is defined by the appended claims. The present disclosure relates to an eye positioning and immobilization device for use during ophthalmological surgery, in particular during glaucoma surgery, pterygium or strabismus surgery. The device offers significant improvements ensuring that the eye is securely positioned and immobilized during surgery, allowing surgeons to perform procedures with greater precision and control. Eye and eye globe are used interchangeably in the present disclosure.

Advantages of the device of the present disclosure include that the device is safer, more practical and highly effective at eye positioning and immobilization during ophthalmological surgery. It allows for more surface area to be exposed for surgery, fine tuning of the positioning of the eye, adjustability and reversibility of direction of the positioning.

The disclosure is thus directed to creating a solid anchor point for an eye traction suture or sutures, namely as to point 2, referred above. The disclosure avoids suture slippage that commonly occurs in the state of art practice that is using tape or a forceps to secure the sutures; allows adjustability of the eye position by means of rotating a fastener or by means where a suture can be twisted; and allows the directing of the suture or sutures in order to provide vector forces on the eye, through for example lateral tabs, for enhancing gaze positions; thus resulting in a broader exposed surface of the eye, in precise exposition and positioning of the surgical area, where the surgeries can be performed. This device is normally to be used in conjunction with one of a multitude of eyelid specula, that accomplishes point 1, referred above, in the most effective way.

It is disclosed an ophthalmic surgical device for eye positioning and immobilization using traction suture or sutures, comprising: a base configured for securing to a periocular region, directly or to surgical drapes covering that same area, wherein the periocular region comprises the inferior periocular area and frontotemporal area, said base having an adhesive surface for adhering to the periocular region; wherein the base comprises an anchoring element for adjustable fixation [i.e. the fixation is variable and reversible so that adjustment can be provided] of the traction suture or sutures. Alternatively, the adhesive surface can be non-adhesive and the adhesive may be applied at a later time before securing the base at the periocular region.

In the disclosed ophthalmic surgical device, the base may have a support edge for supporting [i.e. abutting], externally to the eyelids, against at least a part of a speculum-supported eyelid or against at least a part of an eyelid speculum, such that the eyelid supported by the speculum, or the speculum itself, act as a stopper. The edge must be sufficiently thin, i.e. of a limited thickness, for engaging and supporting against at least a part of a speculum-supported eyelid or against at least a part of an eyelid speculum.

In the disclosed ophthalmic surgical device, the anchoring element is preferably configured for receiving the eye globe traction suture or sutures when the device is adhered to a periocular region by the adhesive surface.

In the disclosed ophthalmic surgical device, the anchoring element may be located in the adhesive surface region on an opposite surface of the base relative to the adhesive surface for securing the base to the periocular region for positioning and immobilization of the eye globe using the traction suture or suture. In particular, the anchoring element may be located directly opposite, relative to the base, to a region comprised in the adhesive surface.

According to the invention, the ophthalmic surgical device for eye globe positioning and immobilization using eye globe traction suture or sutures comprises:
a base configured for securing to a periocular region comprising the inferior periocular and frontotemporal area, said base having an adhesive surface for adhering to the periocular region;
wherein the base comprises an anchoring element for adjustable fixation of the traction suture or sutures;
wherein the anchoring element is configured for receiving the eye globe traction suture or sutures when the device is adhered to a periocular region by the adhesive surface;
wherein the base has a support edge for supporting, externally to the eyelids, against at least a part of a speculum-supported eyelid or against at least a part of an eyelid speculum; and
wherein the anchoring element is located directly opposite, relative to the base, to a region comprised in the adhesive surface.

In an embodiment, the anchoring element comprises a recess [for example, a slot or a slit] for the suture or sutures to pass through and for holding said suture or sutures.

In an embodiment, the anchoring element comprises a rotatable fastener for fastening the suture or sutures; the rotatable fastener being preferably detachable from the anchoring element.

In an embodiment, the rotatable fastener is rotatably coupled to the anchoring element [i.e. the anchoring element serves as a rotation axle of the fastener].

In an embodiment, the attachment of the rotatable fastener and the anchoring element, has a shape for enhancing grasp by pushing the rotatable fastener towards the base, in particular the anchoring element tapering away from the base, for example the anchoring element being conical, for increasing grip when pushing the rotatable fastener towards the base, which results in a progressive and secure tightening of the eye traction suture, that runs in between the rotatable fastener and anchoring element, in opposite directions, preventing its slippage.

In an embodiment, the rotatable fastener comprises a sulcus for the suture or sutures to wind around the rotatable fastener and for holding said suture or sutures.

In an embodiment, the rotatable fastener comprises one or more recesses (or sulcus) for the suture or sutures to pass through and for holding said suture or sutures, in particular the one or more recesses (or sulcus) being arranged on a surface of the rotatable fastener facing said base or facing outwards in respect of the rotatable fastener.

In an embodiment, the anchoring element comprises a detachable, for example clip-on, fastener for fastening the suture or sutures.

In the invention, the base has a support edge for supporting, i.e. abutting, externally to the eyelids, against at least a part of a speculum-supported eyelid or against at least a part of an eyelid speculum.

In an embodiment, the base further comprises two laterally protruding tabs arranged between the support edge and the anchoring element for guiding the one or more sutures, in particular for guiding the one or more sutures towards the orbital rim, further in particular for guiding the one or more sutures towards the inferior orbital rim, in the desired direction.

In an embodiment, the base further comprises a ridge for applying friction to the suture or sutures, and raising the suture or sutures from the base towards a suture running position in the anchoring element or the fastener, if existing.

An embodiment further comprises one or more protrusions between the support edge, including the support edge itself, and the anchoring element for deflecting the one or more sutures.

In an embodiment, the base comprises a plurality of said protrusions wherein the plurality of the protrusions is arranged along an axis between the support edge and the anchoring element.

In an embodiment, the base has a curvature, or curvature and counter curvature, for contouring adjacently the periocular area.

In an embodiment, the base is an elongated base having two ends.

In an embodiment, the base has a narrowing between first and second end.

In an embodiment, the base has two laterally protruding tabs arranged on the narrowed region of the base, or in between extremities of the base.

In an embodiment, the anchoring element is a protruding anchor.

In an embodiment, the anchoring element is an anchor with a hollow volume for passing through and anchoring the traction suture or sutures.

In an embodiment, the rotatable fastener is a rotatable knob, rotatable torniquet or a rotatable screw cap.

In an embodiment, the rotatable fastener comprises one or more levers for rotating the fastener.

In an embodiment, the device is made of a solid material, in particular a polymer, biopolymer, copolymer, metal, or combinations thereof.

In an aspect, the present disclosure relates to an integration, assembly, surgical instrument or kit, comprising an eyelid speculum and the ophthalmic surgical device of any of the embodiments of the present disclosure.

### BRIEF DESCRIPTION OF THE DRAWINGS

The following figures provide preferred embodiments for illustrating the disclosure and should not be seen as limiting the scope of invention.
**Figure 1** shows a perspective view of an embodiment of the disclosed device for eye positioning and immobilization using traction suture or sutures.
**Figure 2** shows a top view of an embodiment of the disclosed device for eye positioning and immobilization using traction suture or sutures.
**Figure 3** shows the front lateral view of a patient having an embodiment of the of the disclosed device for eye positioning and immobilization using traction suture or sutures arranged below the eye and supported against a speculum-supported lower eyelid and/or the speculum itself.
**Figure 4** shows several views of an embodiment of the disclosed device for eye positioning and immobilization using traction suture or sutures with a rotatable fastener, with optional guiding slots in the laterally protruding tabs (Fig. 4B) or without (Fig. 4A).
**Figure 5** shows several views of an embodiment of the disclosed device for eye positioning and immobilization using traction suture or sutures without a rotatable fastener, with optional guiding slots in the laterally protruding tabs (Fig. 5B) or without (Fig. 5A).

### Reference list

**(1)** Base of the ophthalmic surgical device for eye positioning and immobilization
**(2)** Anchoring element
**(3)** Suture deflector(s)
**(4)** Support edge
**(5)** Laterally protruding tabs
**(6)** Ridge
**(7)** Suture
**(8)** Speculum
**(9)** Rotatable fastener
**(10)** Sulcus
**(11)** Guiding slots

### DETAILED DESCRIPTION

The present disclosure relates to an eye positioning and immobilization device for use during ophthalmological surgery, in particular during glaucoma surgery.

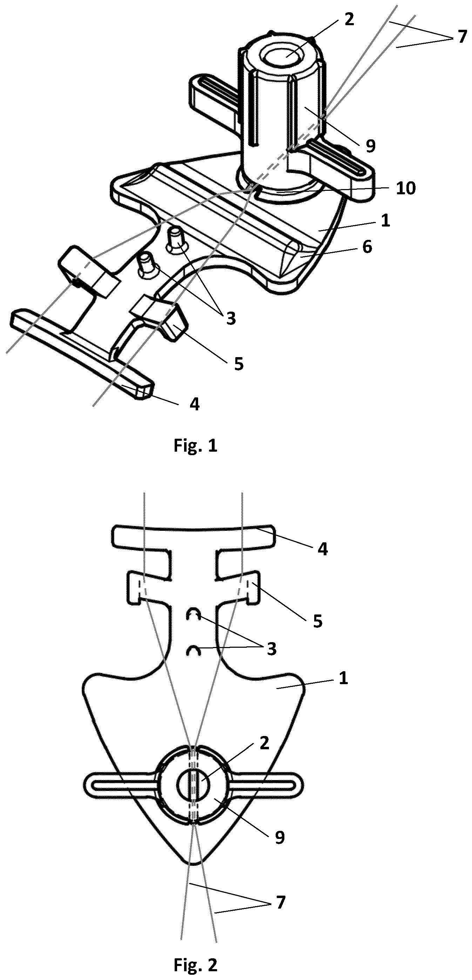

Figure 1 shows a perspective view of an embodiment of the disclosed device for eye positioning and immobilization using traction suture or sutures. Figure 2 shows a top view of an embodiment of the disclosed device for eye positioning and immobilization using traction suture or sutures.

In figure 1 and figure 2, it is disclosed an ophthalmic surgical device for eye positioning and immobilization using traction suture or sutures **7,** comprising:
a base **1** configured for securing to a periocular region comprising the inferior periocular area and frontotemporal area, said base having an adhesive surface for adhering to the periocular region;
wherein the base comprises an anchoring element **2** for adjustable fixation [i.e. the fixation is a reversible so that adjustment can be provided] of the traction suture or sutures.

In an embodiment, the anchoring element **2** comprises a recess [for example, a slot or a slit **10]** for the suture or sutures to pass through and for holding said suture or sutures.

In an embodiment, the anchoring element **2** comprises a rotatable fastener **9** for fastening the suture or sutures.

In an embodiment, the rotatable fastener **9** is rotatably coupled to the anchoring element **2** [i.e. the anchoring element serves as a rotation axle of the fastener].

In an embodiment, the rotatable fastener **9** comprises a sulcus **10** for the suture or sutures to wind around the rotatable fastener and for holding said suture or sutures.

In an embodiment, the rotatable fastener **9** comprises one or more recesses (or sulcus) for the suture or sutures to pass through and for holding said suture or sutures, in particular the one or more recesses (or sulcus) being arranged on a surface of the rotatable fastener facing said base.

In an embodiment, the anchoring element **2** comprises a clip-on fastener for fastening the suture or sutures.

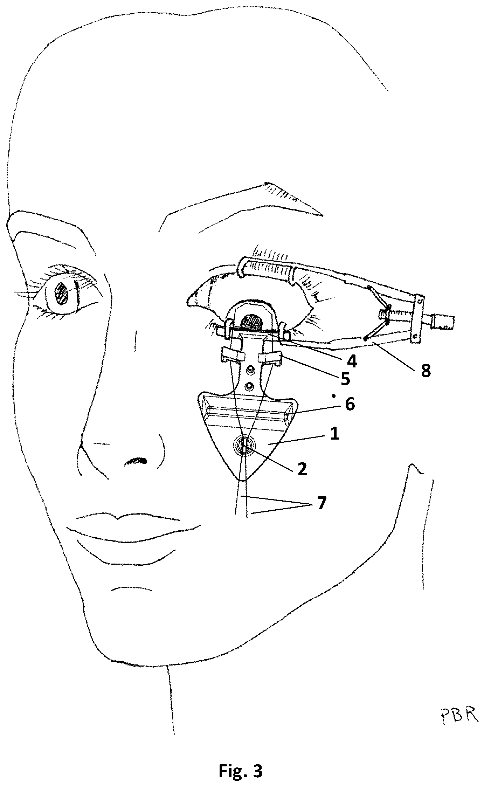

**Figure 3** shows the front-lateral view of a patient having an embodiment of the disclosed device for eye positioning and immobilization using traction suture or sutures arranged below the eye and supported against a speculum-supported lower eyelid and/or the speculum itself.

In **Figures 1-5****,** the base has a support edge **4** for supporting against at least a part of a speculum-supported eyelid or against at least a part of an eyelid speculum.

In an embodiment, the base further comprises two laterally protruding tabs **5** arranged between the support edge and the anchoring element for guiding the one or more sutures towards the periocular area.

In an embodiment, the base further comprises a ridge **6** for applying friction to the suture or sutures, and raising the suture or sutures from the base towards a suture running position in the anchoring element or the fastener, if existing.

An embodiment further comprises one or more protrusions **3** between the support edge and the anchoring element for deflecting the one or more sutures, in particular the base comprising a plurality of said protrusions wherein the plurality of the protrusions are arranged along an axis between the support edge and the anchoring element.

In an embodiment, the base **1** has a curvature, or a curvature and countercurvature, for contouring the periocular area.

In an embodiment, the base **1** is an elongated base having two ends.

In an embodiment, the base **1** has a narrowing between first and second end.

In an embodiment, the base **1** has two laterally protruding tabs **5** arranged on the narrowed region of the base.

In an embodiment, the anchoring element **2** is a protruding anchor.

In an embodiment, the anchoring element **2** is an anchor with a hollow volume for passing suture or sutures through and anchoring the traction suture or sutures.

In an embodiment, the rotatable fastener **9** is a rotatable knob, rotatable torniquet or a rotatable screw cap.

In an embodiment, the rotatable fastener **9** comprises one or more levers for rotating the fastener.

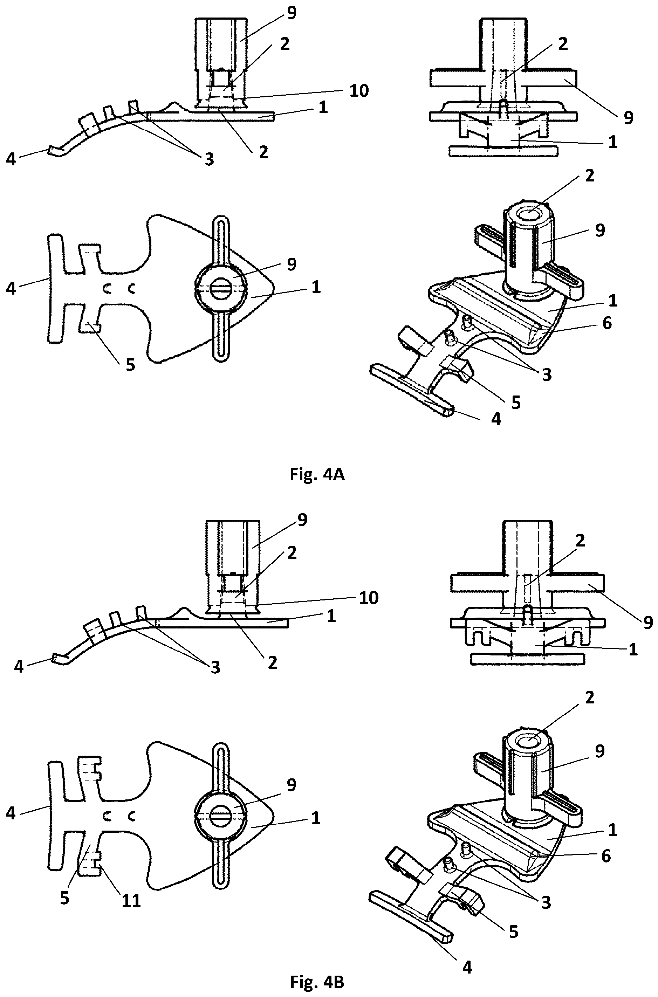

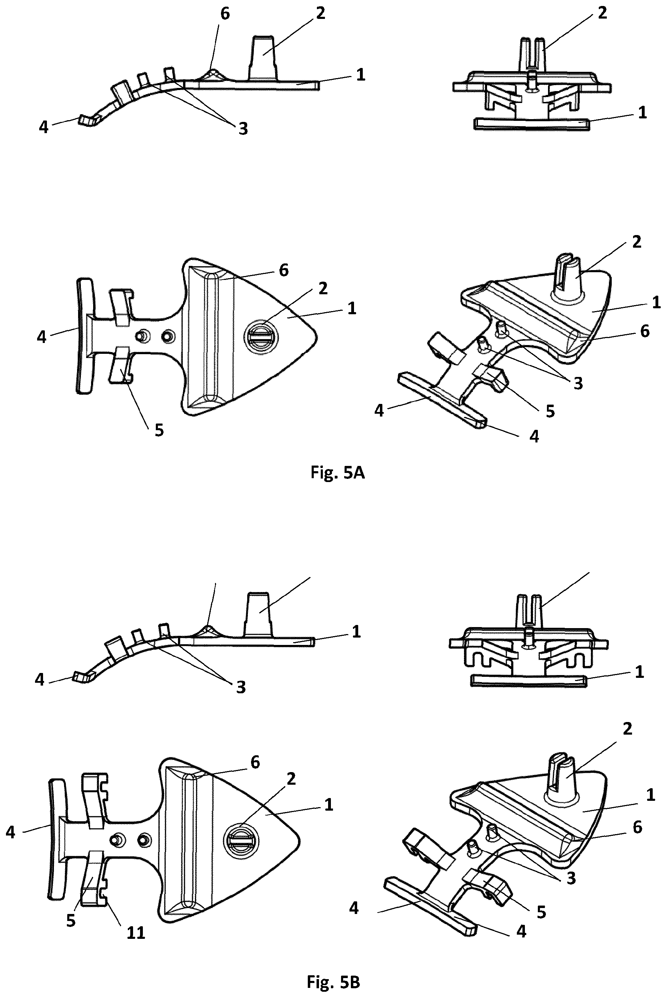

In **figure 4** and **figure 5****,** it is disclosed embodiments of the ophthalmic surgical device for eye positioning and immobilization using traction suture or sutures **7,** with and without, respectively, a rotatable fastener. Figures 3 and 4 show optional guiding slots **11** in the laterally protruding tabs (Fig. 4B, 5B) or without (Fig. 4A, 5A).

The disclosed device can be made of a suitable solid material, a polymer, a biopolymer, a copolymer, metal, or combinations thereof. The embodiments described above are combinable.

## Claims

1. Ophthalmic surgical device for eye globe positioning and immobilization using eye globe traction suture or sutures (7) and for use with an eyelid speculum (8), comprising:
a base (1) configured for securing to a periocular region comprising the inferior periocular and frontotemporal area, said base having an adhesive surface for adhering to the periocular region;
wherein the base comprises an anchoring element (2) for adjustable fixation of the traction suture or sutures;
wherein the anchoring element (2) is configured for receiving the eye globe traction suture or sutures (7) when the device is adhered to a periocular region by the adhesive surface;
wherein the base (1) has a support edge (4) for supporting, externally to the eyelids, against at least a part of a speculum-supported eyelid of the eye or against at least a part of an eyelid speculum (8); and
wherein the anchoring element (2) is located directly opposite, relative to the base (1), to a region comprised in the adhesive surface.

2. Ophthalmic surgical device for eye globe positioning and immobilization according to the previous claim wherein the base (1) further comprises two laterally protruding tabs (5) arranged between the support edge (4) and the anchoring element (2) for guiding the one or more sutures (7), in particular the two laterally protruding tabs (5) being arranged for guiding the one or more sutures (7) in a needed direction and towards the orbital rim.

3. Ophthalmic surgical device for eye globe positioning and immobilization according to any of the previous claims wherein the anchoring element (2) comprises a recess for the suture or sutures (7) to pass through and for holding said suture or sutures (7).

4. Ophthalmic surgical device for eye globe positioning and immobilization according to any of the previous claims wherein the anchoring element (2) comprises a rotatable fastener (9) for fastening the suture or sutures (7), in particular wherein the rotatable fastener (9) is rotatably coupled to the anchoring element (2).

5. Ophthalmic surgical device for eye globe positioning and immobilization according to the previous claim wherein the rotatable fastener (9) comprises a sulcus (10) for the suture or sutures (7) to wind around the rotatable fastener (9) and for holding said suture or sutures (7), in particular wherein the rotatable fastener (9) comprises one or more recesses for the suture or sutures (7) to pass through and for holding said suture or sutures (7).

6. Ophthalmic surgical device for eye globe positioning and immobilization according to any of the previous claims wherein the base (1) further comprises a ridge (6) for applying friction to the suture or sutures (7), and raising the suture or sutures (7) from the base (1) towards a suture running position in the anchoring element (2) or the fastener, if existing.

7. Ophthalmic surgical device for eye globe positioning and immobilization according to any of the previous claims, wherein the base (1) further comprises one or more protrusions (3) between the support edge (4), including the support edge itself, and the anchoring element (2) for deflecting the one or more sutures (7).

8. Ophthalmic surgical device for eye globe positioning and immobilization according to any of the previous claims wherein the base (1) has a curvature, or curvature and counter curvature for contouring adjacently the periocular area.

9. Ophthalmic surgery device for eye globe positioning and immobilization according to any of the previous claims, wherein the base (1) is an elongated base having two ends, in particular the base having a narrowing between first and second end, further in particular wherein the base (1) further comprises two laterally protruding tabs (5) arranged on the narrowed region of the base (1) and arranged between the support edge (4) and the anchoring element (2) for guiding the one or more sutures (7).

10. Ophthalmic surgical device for eye globe positioning and immobilization according to any of the previous claims wherein the anchoring element (2) is a protruding anchor, particular comprising a fastener (9) with a matching hollow volume for coupling with said protruding anchor to anchor said suture or sutures (7), further in particular shaped to anchor suture(s) fastened in opposite slip directions for reducing suture slippage.

11. Ophthalmic surgical device for eye globe positioning and immobilization according to any of the previous claims wherein the anchoring element (2) is an anchor with a hollow volume for passing through and anchoring the traction suture or sutures (7), in particular comprising a fastener with a matching protrusion for coupling with said hollow volume to anchor and lock said suture or sutures (7), further in particular shaped to anchor suture(s) fastened in opposite slip directions for reducing suture slippage.

12. Ophthalmic surgical device for eye globe positioning and immobilization according to any of the previous claims, wherein the anchoring element (2) is shaped to taper away from the base (1), for increasing grip when the fastener is pushed towards the base (1), in particular the anchoring element (2) being conical.

13. Ophthalmic surgical device for eye globe positioning and immobilization according to claim 4, claim 5, or any of the claims 6-12 when dependent on claim 4 or 5, wherein the rotatable fastener (9) is a rotatable knob, rotatable torniquet or a rotatable screw cap, and/or wherein the rotatable fastener comprises one or more levers for assisting on rotation of the fastener.

14. Ophthalmic surgical device for eye globe positioning and immobilization according to any of the previous claims wherein the device is made of a solid material, in particular a polymer, biopolymer, copolymer, metal, or combinations thereof.

15. Integration, assembly, surgical instrument or kit, comprising an eyelid speculum (8) and the ophthalmic surgical device according to any of the previous claims.

## Patentansprüche

1. Ophthalmologische chirurgische Vorrichtung zur Positionierung und Immobilisierung des Augapfels unter Verwendung einer oder mehrerer Augapfel-Zug-Nähte (7) und zur Verwendung mit einem Augenlidspekulum (8), umfassend:
eine Basis (1), die zur Befestigung an einem periokularen Bereich konfiguriert ist, der den unteren periokularen und frontotemporalen Bereich umfasst, wobei die Basis eine Klebefläche zum Anhaften an dem periokularen Bereich aufweist;
wobei die Basis ein Verankerungselement (2) zur einstellbaren Fixierung des oder der Zug-Nähte (7) umfasst;
wobei das Verankerungselement (2) so konfiguriert ist, dass es des oder der Augapfel-Zug-Nähte (7) aufnimmt, wenn die Vorrichtung mit der Klebefläche an einen periokularen Bereich haftet;
wobei die Basis (1) einen Stützrand (4) zum Stützen außerhalb der Augenlider gegen mindestens einen Teil eines spekulumgestützten Augenlids des Auges oder gegen mindestens einen Teil eines Augenlidspekulums (8) aufweist; und
wobei das Verankerungselement (2) in Bezug auf die Basis (1) direkt gegenüber einem Bereich angeordnet ist, der in der Klebefläche enthalten ist.

2. Ophthalmologische chirurgische Vorrichtung zur Positionierung und Immobilisierung des Augapfels nach dem vorhergehenden Anspruch, wobei die Basis (1) ferner zwei seitlich vorstehende Laschen (5) umfasst, die zwischen dem Stützrand (4) und dem Verankerungselement (2) angeordnet sind, um die eine oder mehreren Nähte (7) zu führen, wobei insbesondere die beiden seitlich vorstehenden Laschen (5) so angeordnet sind, dass sie die eine oder mehreren Nähte (7) in einer benötigten Richtung und zum Orbitalrand hin führen.

3. Ophthalmologische chirurgische Vorrichtung zur Positionierung und Immobilisierung des Augapfels nach einem der vorhergehenden Ansprüche, wobei das Verankerungselement (2) eine Aussparung zum Durchführen der Naht oder Nähte (7) und zum Halten der Naht oder Nähte (7) aufweist.

4. Ophthalmologische chirurgische Vorrichtung zur Positionierung und Immobilisierung des Augapfels nach einem der vorhergehenden Ansprüche, wobei das Verankerungselement (2) ein drehbares Befestigungselement (9) zur Befestigung der Naht oder Nähte (7) umfasst, wobei insbesondere das drehbare Befestigungselement (9) drehbar mit dem Verankerungselement (2) gekoppelt ist.

5. Ophthalmologische chirurgische Vorrichtung zur Positionierung und Immobilisierung des Augapfels nach dem vorhergehenden Anspruch, wobei das drehbare Befestigungselement (9) einen Sulcus (10) für der Naht oder Nähte (7) aufweist, die sich um das drehbare Befestigungselement (9) wickeln und der Naht oder Nähte (7) halten, insbesondere wobei das drehbare Befestigungselement (9) eine oder mehrere Aussparungen zum Durchführen der Naht oder Nähte (7) und zum Halten der Naht oder Nähte (7) umfasst.

6. Ophthalmologische chirurgische Vorrichtung zur Positionierung und Immobilisierung des Augapfels nach einem der vorhergehenden Ansprüche, wobei die Basis (1) ferner eine Rippe (6) zum Ausüben von Reibung auf die Naht oder Nähte (7) und zum Anheben der Naht oder Nähte (7) von der Basis (1) in Richtung einer Nahtverlaufsposition in dem Verankerungselement (2) oder dem Befestigungselement, falls vorhanden, umfasst.

7. Ophthalmologische chirurgische Vorrichtung zur Positionierung und Immobilisierung des Augapfels nach einem der vorhergehenden Ansprüche, wobei die Basis (1) ferner einen oder mehrere Vorsprünge (3) zwischen dem Stützrand (4), einschließlich des Stützrandes selbst, und dem Verankerungselement (2) zur Umlenkung des einen oder der mehreren Nähte (7) umfasst.

8. Ophthalmologische chirurgische Vorrichtung zur Positionierung und Immobilisierung des Augapfels nach einem der vorhergehenden Ansprüche, wobei die Basis (1) eine Krümmung oder eine Krümmung und eine Gegenkrümmung aufweist, um den periokularen Bereich angrenzend zu konturieren.

9. Ophthalmologische chirurgische Vorrichtung zur Positionierung und Immobilisierung des Augapfels nach einem der vorhergehenden Ansprüche, wobei die Basis (1) eine längliche Basis mit zwei Enden ist, insbesondere die Basis eine Verengung zwischen dem ersten und dem zweiten Ende aufweist, wobei die Basis (1) ferner insbesondere zwei seitlich abstehende Laschen (5) umfasst, die an dem verengten Bereich der Basis (1) angeordnet sind und zwischen dem Stützrand (4) und dem Verankerungselement (2) zur Führung der einen oder mehreren Nähte (7) angeordnet sind.

10. Ophthalmologische chirurgische Vorrichtung zur Positionierung und Immobilisierung des Augapfels nach einem der vorhergehenden Ansprüche, wobei das Verankerungselement (2) ein vorstehender Anker ist, der insbesondere ein Befestigungselement (9) mit einem passenden Hohlraum zur Kopplung mit dem vorstehenden Anker umfasst, um die Naht oder Nähte (7) zu verankern, und ferner insbesondere so geformt ist, dass es in entgegengesetzten Gleitrichtungen befestigte Nähte verankert, um ein Verrutschen der Nähte zu verringern.

11. Ophthalmologische chirurgische Vorrichtung zur Positionierung und Immobilisierung des Augapfels nach einem der vorhergehenden Ansprüche, wobei das Verankerungselement (2) ein Anker mit einem Hohlraum zum Durchführen und Verankern des oder der Zug-Nähte (7) ist, der insbesondere ein Befestigungselement mit einem passenden Vorsprung zur Kopplung mit dem Hohlraum umfasst, um der Naht oder Nähte (7) zu verankern und zu verriegeln, das ferner insbesondere so geformt ist, dass es in entgegengesetzten Gleitrichtungen befestigte Nähte verankert, um ein Verrutschen der Nähte zu verringern.

12. Ophthalmologische chirurgische Vorrichtung zur Positionierung und Immobilisierung des Augapfels nach einem der vorhergehenden Ansprüche, wobei das Verankerungselement (2) so geformt ist, dass es sich von der Basis (1) weg verjüngt, um den Halt zu erhöhen, wenn das Befestigungselement in Richtung der Basis (1) gedrückt wird, wobei das Verankerungselement (2) insbesondere konisch ist.

13. Ophthalmologische chirurgische Vorrichtung zur Positionierung und Immobilisierung des Augapfels nach Anspruch 4, Anspruch 5 oder einem der Ansprüche 6 bis 12, wenn sie von Anspruch 4 oder 5 abhängt, wobei das drehbare Befestigungselement (9) ein drehbarer Knopf, ein drehbares Torniquet oder eine drehbare Schraubkappe ist, und/oder wobei das drehbare Befestigungselement einen oder mehrere Hebel zur Unterstützung der Drehung des Befestigungselements umfasst.

14. Ophthalmologische chirurgische Vorrichtung zur Positionierung und Immobilisierung des Augapfels nach einem der vorhergehenden Ansprüche, wobei die Vorrichtung aus einem festen Material, insbesondere einem Polymer, Biopolymer, Copolymer, Metall oder Kombinationen davon, hergestellt ist.

15. Integration, Baugruppe, chirurgisches Instrument oder Kit, umfassend ein Augenlidspekulum (8) und die ophthalmologische chirurgische Vorrichtung nach einem der vorhergehenden Ansprüche.

## Revendications

1. Dispositif chirurgical ophtalmique pour le positionnement et l'immobilisation du globe oculaire à l'aide d'une ou plusieurs sutures (7) de traction du globe oculaire et destiné à être utilisé avec un spéculum palpébral (8), comprenant :
une base (1) configurée pour être fixée à une région périoculaire comprenant la zone périoculaire inférieure et la zone frontotemporale, ladite base ayant une surface adhésive pour adhérer à la région périoculaire ;
dans lequel la base comprend un élément d'ancrage (2) pour la fixation réglable de la suture ou des sutures de traction ;
dans lequel l'élément d'ancrage (2) est configuré pour recevoir la suture ou les sutures (7) de traction du globe oculaire lorsque le dispositif est adhéré à une région périoculaire par la surface adhésive ;
dans lequel la base (1) a un bord d'appui (4) destiné à s'appuyer, à l'extérieur des paupières, contre au moins une partie d'une paupière soutenue par un spéculum ou contre au moins une partie d'un spéculum palpébral (8) ; et
dans lequel l'élément d'ancrage (2) est situé directement en face, par rapport à la base (1), d'une région comprise dans la surface adhésive.

2. Dispositif chirurgical ophtalmique pour le positionnement et l'immobilisation du globe oculaire selon la revendication précédente, dans lequel la base (1) comprend en outre deux languettes (5) saillant latéralement, disposées entre le bord d'appui (4) et l'élément d'ancrage (2) pour guider la suture ou les sutures (7), en particulier les deux languettes (5) saillantes latérales étant disposées pour guider la suture ou les sutures (7) dans une direction nécessaire et vers le rebord orbitaire.

3. Dispositif chirurgical ophtalmique pour le positionnement et l'immobilisation du globe oculaire selon l'une quelconque des revendications précédentes, dans lequel l'élément d'ancrage (2) comprend un évidement pour le passage de la suture ou des sutures (7) et pour maintenir ladite suture ou lesdites sutures (7).

4. Dispositif chirurgical ophtalmique pour le positionnement et l'immobilisation du globe oculaire selon l'une quelconque des revendications précédentes, dans lequel l'élément d'ancrage (2) comprend une fixation rotative (9) pour fixer la suture ou les sutures (7), en particulier dans lequel la fixation rotative (9) est couplée de manière rotative à l'élément d'ancrage (2).

5. Dispositif chirurgical ophtalmique pour le positionnement et l'immobilisation du globe oculaire selon la revendication précédente, dans lequel la fixation rotative (9) comprend un sillon (10) pour que la suture ou les sutures (7) s'enroulent autour de la fixation rotative (9) et pour maintenir ladite suture ou lesdites sutures (7), en particulier dans lequel la fixation rotative (9) comprend un ou plusieurs évidements pour que la suture ou les sutures (7) puissent passer à travers et pour maintenir ladite suture ou lesdites sutures (7).

6. Dispositif chirurgical ophtalmique pour le positionnement et l'immobilisation du globe oculaire selon l'une quelconque des revendications précédentes, dans lequel la base (1) comprend en outre une arête (6) pour exercer une friction sur la suture ou les sutures (7) et soulever la suture ou les sutures (7) de la base (1) vers une position de suture dans l'élément d'ancrage (2) ou la fixation, le cas échéant.

7. Dispositif chirurgical ophtalmique pour le positionnement et l'immobilisation du globe oculaire selon l'une quelconque des revendications précédentes, dans lequel la base (1) comprend en outre une ou plusieurs saillies (3) entre le bord d'appui (4), y compris le bord d'appui lui-même, et l'élément d'ancrage (2) pour dévier la suture ou les sutures (7).

8. Dispositif chirurgical ophtalmique pour le positionnement et l'immobilisation du globe oculaire selon l'une quelconque des revendications précédentes, dans lequel la base (1) présente une courbure, ou une courbure et une contre-courbure, pour contourner la zone périoculaire de manière adjacente.

9. Dispositif chirurgical ophtalmique pour le positionnement et l'immobilisation du globe oculaire selon l'une quelconque des revendications précédentes, dans lequel la base (1) est une base allongée avec deux extrémités, en particulier la base présentant un rétrécissement entre la première et la deuxième extrémité, plus en particulier dans lequel la base (1) comprend en outre deux languettes (5) saillantes latérales disposées sur la région rétrécie de la base (1) et disposées entre le bord d'appui (4) et l'élément d'ancrage (2) pour guider la suture ou les sutures (7).

10. Dispositif chirurgical ophtalmique pour le positionnement et l'immobilisation du globe oculaire selon l'une quelconque des revendications précédentes, dans lequel l'élément d'ancrage (2) est un ancrage saillant, comprenant en particulier une fixation (9) avec un volume creux correspondant pour le couplage avec ledit ancrage saillant afin d'ancrer ladite suture ou lesdites sutures (7), plus en particulier formé pour ancrer une ou plusieurs sutures fixées dans des directions de glissement opposées, afin de réduire le glissement de la suture.

11. Dispositif chirurgical ophtalmique pour le positionnement et l'immobilisation du globe oculaire selon l'une quelconque des revendications précédentes, dans lequel l'élément d'ancrage (2) est un ancrage avec un volume creux pour faire passer et ancrer la suture ou les sutures (7) de traction, comprenant en particulier une fixation avec une saillie correspondante pour s'accoupler avec ledit volume creux afin d'ancrer et de bloquer ladite suture ou lesdites sutures (7), et en particulier formé pour ancrer une ou plusieurs sutures fixées dans des directions de glissement opposées afin de réduire le glissement de la suture.

12. Dispositif chirurgical ophtalmique pour le positionnement et l'immobilisation du globe oculaire selon l'une quelconque des revendications précédentes, dans lequel l'élément d'ancrage (2) est formé de manière à s'effiler à partir de la base (1), afin d'augmenter la prise lorsque la fixation est poussée vers la base (1), l'élément d'ancrage (2) étant en particulier de forme conique.

13. Dispositif chirurgical ophtalmique pour le positionnement et l'immobilisation du globe oculaire selon selon la revendication 4, la revendication 5 ou l'une quelconque des revendications 6 à 12 lorsqu'elles dépendent de la revendication 4 ou 5, dans lequel la fixation rotative (9) est un bouton rotatif, un garrot rotatif ou un bouchon à vis rotatif, et/ou dans lequel la fixation rotative comprend un ou plusieurs leviers pour faciliter la rotation de la fixation.

14. Dispositif chirurgical ophtalmique pour le positionnement et l'immobilisation du globe oculaire selon l'une quelconque des revendications précédentes, dans lequel le dispositif est fabriqué à partir d'un matériau solide, en particulier un polymère, un biopolymère, un copolymère, un métal, ou une combinaison de ceux-ci.

15. Intégration, assemblage, instrument chirurgical ou kit comprenant un spéculum palpébral (8) et le dispositif chirurgical ophtalmique selon l'une quelconque des revendications précédentes.
